# EUROPEAN PATENT SPECIFICATION

(11) **EP 4 233 826 B1**
(45) Date of publication and mention of the grant of the patent: **26.03.2025**
(21) Application number: 23171430.4
(22) Date of filing: 09.11.2020
(51) Int. Cl.: A61J 1/20, A61J 3/00, A61M 5/14

(54) **PISTON VALVING FOR SERIALLY CONNECTABLE DRUG MODULES OF A COMBINATORIAL DRUG DELIVERY DEVICE**
KOLBENVENTIL FÜR SERIELL VERBINDBARE ARZNEIMITTELMODULE EINER KOMBINATORISCHEN ARZNEIMITTELABGABEVORRICHTUNG
SOUPAPE À PISTON POUR MODULES DE MÉDICAMENT POUVANT ÊTRE CONNECTÉS EN SÉRIE D'UN DISPOSITIF D'ADMINISTRATION DE MÉDICAMENT COMBINATOIRE

(30) Priority: 08.11.2019 US 201962933045 P
(43) Date of publication of application: 30.08.2023
(62) Divisional of application: 20816827.8
(73) Proprietor: Bristol-Myers Squibb Company, Princeton, NJ 08543 (US)
(72) Inventor: MCLOUGHLIN, Martin John, Princeton, 08543 (US); ZIEMINSKI, Stephen Lawrence, Princeton, 08543 (US); HOWANSKY, Mark Steven, Princeton, 08543 (US); MUMPOWER, Mariano, Baltimore, 21230 (US); SPRINGER, Melanie Marie, Baltimore, 21230 (US); GOETZ, Katherine Alina, Baltimore, 21230 (US); LANE, Benjamin Richard, Baltimore, 21230 (US)
(74) Representative: White, Andrew John

(56) References cited:
- WO-A1-2016/205687
- WO-A2-02/102295
- WO-A2-2007/107406

## Description

### Field of the Invention

The field of the present invention is the compounding and preparation of liquid drugs, especially for intra-venous infusion and direct patient infusion. More particularly the invention relates to a sealing mechanism for devices used in the preparation and compounding of combinations of two or more drugs.

### Background to the Invention

It is common practice in the administration of drugs by intravenous infusion for the drugs to be compounded within a pharmacy environment. Such drugs are typically supplied sterile in glass vials and may be supplied in solid or aqueous solution form. When supplied in solid form the drugs must be reconstituted with a sterile aqueous diluent prior to transfer to the infusion bag. The person skilled in the art will appreciate that such drug formulations will typically include several excipients for example buffers, pH modifiers, tonicity modifiers, stabilizers and so on. Typically liquid drugs for intra-venous infusion are compounded in an infusion bag in a pharmacy environment prior to transfer to the patient for infusion. Because of the need to maintain sterility of the drugs while compounding the compounding procedure is typically performed in an aseptic pharmacy hood. Typically, the pharmacist or pharmacy technician (practitioner) will prepare the drugs in accordance with an individual patient prescription.

After ensuring the hood is clear of all materials the practitioner will retrieve vials of the drugs required per the prescription from the pharmacy stocks and will verify their identity and strength. The verification process may be assisted by use of a bar code scanner or other identification technology. The practitioner will also pick from stock all of the other necessary equipment required to safely prepare the drugs for infusion including the infusion bag itself, syringes, needles, transfer sets, gloves, sharps disposal containers and so on. Once all of the necessary equipment has been assembled the practitioner will follow a protocol for the preparation of the drugs which may include the reconstitution of solid drugs by addition of diluents, the ordered withdrawal of liquid drugs from their individual vials into the IV bag via the transfer port. Typically this procedure is performed manually and involves the use of multiple needles. The risk of needle-stick injuries to the practitioner is increased by each needle required to effect the compounding of the drugs. With high potency or toxicity drugs, e.g. cytotoxic agents for chemotherapy, this presents a considerable exposure risk for the practitioner.

To eliminate some of the risks associated with manual preparation including exposure to dangerous drugs and the risk of medication errors, pharmacy compounding machines are known to the person skilled in the art which automate many of the steps involved in the preparation and compounding of drugs. Typically such machines are complex electromechanical systems which implement sophisticated precision dispensing mechanisms for the accurate reconstitution of liquid drugs. Aside from their cost, size and complexity, many of the designs for such machines described in the art draw liquid drugs from a stock reservoir and so only use a fraction of the drug in the container. Because of the need to maintain sterility, unused drug solutions must typically be discarded and so are wasted. With the very high cost of some drugs, especially biologic drugs, this waste is a significant undesirable cost. When the wasted drugs are cytotoxic agents, their disposal creates a significant environmental and safety hazard.

Recent advances in medicine, particularly in the treatment of cancer, have demonstrated that therapeutically beneficial effects can be achieved by the synergistic combination of two or more drugs.

For example, recent clinical research has demonstrated that the combination of an anti-PD-1 checkpoint inhibitor drug with a CTLA4 checkpoint inhibitor can have beneficial synergistic effects in some tumor types which can lead to better clinical outcomes than could be achieved by the individual administration of either drug alone. Typically such checkpoint inhibitor drugs are biotechnology derived monoclonal antibodies or fragments thereof of the immunoglobulin type. In some situations it may be beneficial to combine such biologic drugs with conventional chemotherapy agents such as cytotoxic drugs.

Through the utilization of serially connectable drug modules as described in applicant's co-pending applications (U.S. Provisional Patent Appl. No. 62/670,266, filed May 11, 2018; PCT Appl. No. PCT/US2019/031727, filed May 10, 2019; PCT Appl. No. PCT/2019/031762, filed May 10, 2019; and, PCT Appl. No. PCT/US2019/031791, filed May 10, 2019) combinations of drugs can be successfully stored, shipped, and administered to patients and a manner that allows for sufficient flexibility while simultaneously minimizing product waste. These drug modules utilize common off-the-shelf vial primary containers, which during administration are pierced with a spike located inside the module, which allows the liquid drug within the vial to enter the internal fluidics of the module. It is critical that a sealing mechanism be present within the fluidics of the module that is not only able to contain the liquid drug product within the module fluidics during the spiking process, but also have the capability to open the fluidic path during use of the product to allow the drug product to flow. As this product is envisioned to be disposable, the ideal sealing mechanism must be low in cost to produce while also being extremely reliable and repeatable.

Applicant has now realized that the combinatorial principles described in U.S. Provisional Patent Application No. 62/670,266, filed on May 11, 2018, PCT Appl. No. PCT/US2019/031727, filed May 10, 2019, PCT Appl. No. PCT/2019/031762, filed May 10, 2019, and, PCT Appl. No. PCT/US2019/031791, filed May 10, 2019, to the same assignee as herein, can address several of the challenges encountered in the preparation and compounding of drugs for intra-venous infusion and can provide several advantages including but not limited to simplification of pharmacy procedures, reduction in the risk of medication errors, containment and protection for the practitioner from highly potent or highly toxic agents, reduction in the risk of needle-stick injuries, reduction or elimination of drug waste, avoidance of the need for complex and expensive pharmacy compounding machines. As a consequence of these advantages in embodiments the present invention may further enable the preparation and compounding of drugs for IV infusion at locations remote from the pharmacy, and by a non-specialist practitioner, for example by a suitably trained technician or nurse at the patient's home. This possibility is enhanced by the intrinsic portability of the system described herein.

WO2016/205687 describes a device that is provided for pooling a fluid from a container unit having at least one container, and that includes an inlet port having at least one inlet channel configured for receiving the fluid or ambient air, and an outlet port having at least one outlet channel configured for delivering the fluid to an attachment. Both inlet and outlet ports are disposed on the device. A cavity is provided for accommodating insertion of the container unit for pooling the fluid from the at least one container. At least one spike is disposed in the cavity and configured for puncturing a stopper of the at least one container when the container unit transitions from an upper position to a lower position.

### Summary of the Invention

Aspects of the invention are as set out in the appended claims. According to the present disclosure, for modules useable with a combinatorial drug delivery device, a moveable piston seal with a bypass chamber put inline of the module's outlet fluidics provides a means of sealing the module's fluidics from atmosphere during vial piercing, while is also able to allow the fluid path to open when vacuum is applied to the outer face of the piston seal.

Two lumen pathways, inlet and outlet, within the spike that enters the vial's drug chamber, divide the module's fluidic pathway. The inlet allows the liquid drug product from the preceding connected module to enter the spiked vial, while the outlet path moves fluid from the vial to the proceeding module. A spring-loaded seal at the entrance of the inlet path is used to interface with preceding modules, this seal ensures the pathway is only opened at the time of connection with a module, therefore, once the vial is spiked, the inlet pathway is closed to the atmosphere and the vial contents cannot escape.

During vial spike, the volume of the spike entering the vial displaces some of the fluid within the vial causing slight pressurization of the vial's contents, which forces it into the spike lumens. On the inlet side this pressure is contained by a spring-loaded seal, on the outlet side the fluid enters the spike lumen and is sealed by a piston seal. Any pressure during the spiking process will slightly push the piston seal allowing the liquid chamber to increase volumetrically, thus reducing the pressure from spiking. On the opposite side of the piston seal is the outlet to the proceeding module's inlet. A small bypass is cut into the module body next to the piston seal connecting the outlet chamber to the outlet fitting. When a source of vacuum is applied to the outlet fitting this will cause the piston to move, exposing the bypass port to the vial's fluidic pathway thus opening up a path way for fluid to travel from the vial to the outlet fitting.

### Brief Description of the Drawings

Figure 1 shows a piston valve in a closed position in accordance with the subject invention;
Figure 2 shows a piston valve relieving vial positive pressure in accordance with the subject invention;
Figure 3 shows a piston valve in an open position in accordance with the subject invention;
Figure 4 shows a drug vial coupled to valving in accordance with the subject invention;
Figure 5 shows piston valves connected in series in accordance with the subject invention; and,
Figure 6 shows a fluid pathway for piston valves connected in series in accordance with the subject invention.

### Detailed Description

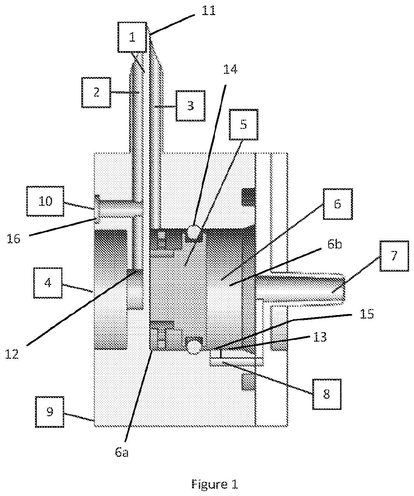

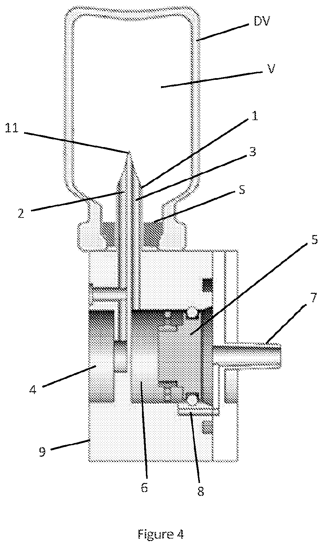

The subject invention is particularly well-suited for use with serially-connected drug modules, particularly in forming fluid paths therebetween. The subject invention is shown in a context of a single module 9, but it is understood that the subject invention operates with a plurality of similarly formed modules 9, serially connected. As depicted in Figures 1 and 4, each of the modules 9 includes valving having a vial spike 1 used to pierce a vial septum S extending into the interior volume V of a liquid filled drug vial DV. A free, distal end 11 of the vial spike 1 may be sharpened to facilitate piercing of the vial septum S. The vial spike 1 must be provided with sufficient length to fully pierce the septum S in accessing the interior volume V.

The vial spike 1 includes two lumens dividing the module fluidics into separate circuits, an inlet path 2, and an outlet path 3. With the vial spike 1 piercing the septum S, both the inlet path 2 and the outlet path 3 are open at the free end 11 of the vial spike 1 and in communication with the interior volume V of the drug vial DV. The inlet path 2 extends from the interior volume V of the drug vial DV to an inlet opening 12 which is selectively sealed by spring-biased sealing port 4. A vent 10 exists on the inlet path 2, preferably between the free end 11 and the inlet opening 12, to allow air into the drug vial DV, as needed, to displace fluid during transfer. Preferably, the vent 10 is a one-way vent which is normally closed and allows for gas flow into the inlet path 2. The outlet path 3 extends from the interior volume V of the drug vial DV down to outlet chamber 6. A piston valve 5 is slidably seated inside the outlet chamber 6 creating a seal against the chamber inner wall 13. The piston valve 5 may include a radial seal 14 in sealing contact with the wall 13 to define the seal whilst allowing the piston valve 5 to slide within the outlet chamber 6.

The piston valve 5 forms a seal in the outlet chamber 6 to define first and second chamber portions 6a, 6b, which are adjustable in size with movement of the piston valve 5 within the outlet chamber 6, with the seal therebetween being maintained. The outlet path 3 is in communication with the first chamber portion 6a. An outlet fitting 7 is provided which is in communication with the outlet chamber 6, particularly, the second outlet chamber 6b.

In an initial state, as shown in Figure 1, the piston valve 5 is placed in a first position at the medial end of the outlet chamber 6 preventing fluid entering from the outlet lumen 3 from accessing the outlet fitting 7. A bypass passageway 8 connects the outlet chamber 6 to the outlet fitting 7. In particular, the bypass passageway 8 terminates at an opening 15 in the wall 13 of the outlet chamber 6. With the piston valve 5 in the first position, the first chamber portion 6a is sealed from the bypass passageway 8.

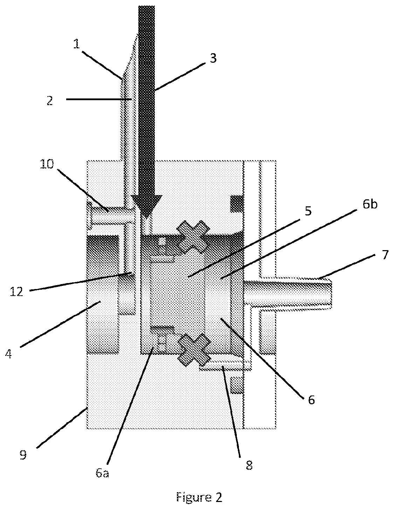

With this arrangement (the piston valve 5 being in the first position), and, as shown in Figure 2, pressurized fluid forced from the drug vial DV, as a result of vial spike 1 spiking the septum S, will be contained in the inlet pathway 2 by the spring-biased sealing port 4, while any entering the outlet pathway 3 will be contained within the first chamber portion 6a behind the piston valve 5. As shown in Figure 2, pressure in the fluid may move the piston valve 5 slightly; this movement in position will equilibrate the pressure within the drug vial DV and fluid path to a negligible amount.

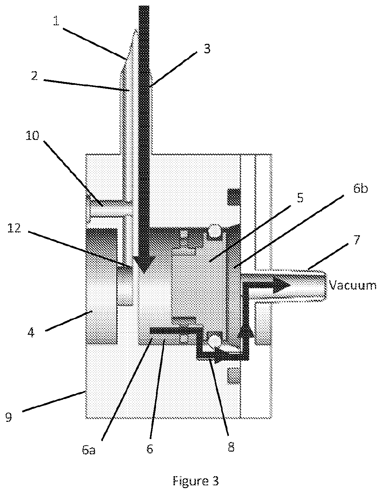

As shown in Figure 3, at the time of fluid transfer, a source of negative pressure, e.g., a vacuum, will be provided to the outlet fitting 7, thereby evacuating air from the bypass passageway 8 and the outlet chamber 6, particularly, the second chamber portion 6b. This will generate a pressure differential across the piston valve 5 which will cause the piston valve 5 to slide along the outlet chamber 6 towards the outlet fitting 7. This results in an increase in volume of the first chamber portion 6a. Eventually, as the piston valve 5 continues moving it will pass over the bypass passageway 8, thereby allowing the first chamber portion 6a, along with the outlet path 3, to come into communication with the bypass passageway 8. As shown in Figure 3, with the bypass passageway 8 carrying vacuum, fluid will then be able to move from the vial, through the outlet pathway 3, through the bypass passageway 8 circumventing the piston valve 5, and out through the outlet fitting 7.

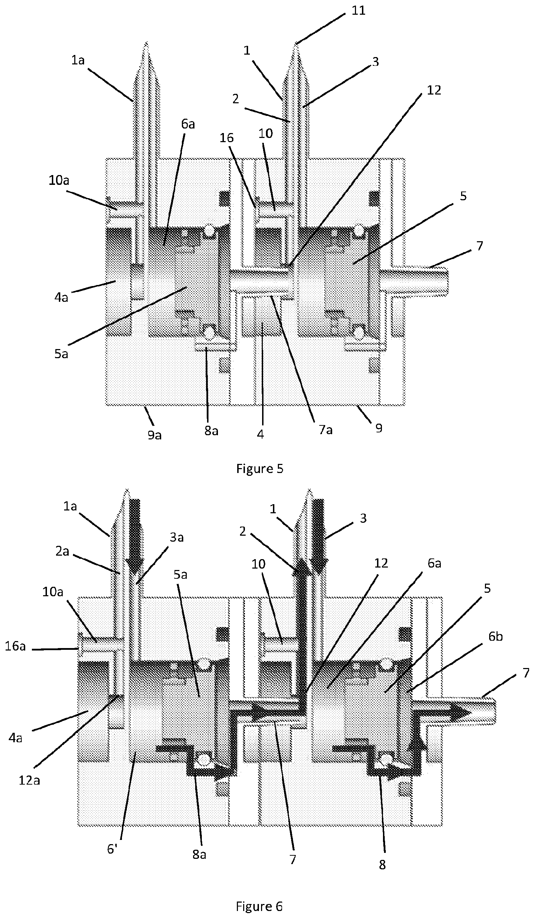

As shown in Figures 5 and 6, a plurality of the modules 9 may be coupled in series with the outlet fitting 7a of a secondary module 9a breaching the sealing port 4 of an adjacent module 9 such that the outlet fitting 7a is in communication with the inlet opening 12 thereof. The secondary module 9a is formed similar to the module 9 with like parts being similarly numbered, but additionally designated with the letter "a" (except for the outlet chamber of the secondary module which is designated as 6'). The coupled arrangement allows for a fluid path to be defined from a drug vial DV coupled to vial spike 1a of the secondary module 9a to the outlet fitting 7 of the module 9, through a drug vial DV coupled to vial spike 1, as shown schematically in Figure 6. This fluid pathway is achieved with sliding movement of piston valve 5a, resulting from negative pressure being applied to the outlet fitting 7a, via the outlet path 3 and the inlet path 2 of the module 9. In similar manner to that described above, flow bypasses the piston valve 5a with the bypass passageway 8a, upon sufficient sliding displacement of the piston valve 5a. This arrangement allows for a series of modules 9 to be coupled, with the respective drug vials DV being in-line to define a single flow path, acted upon by a single source of negative pressure.

It is also noted that the vent 10 is located to terminate at a vent opening 16 located to be exposed to open atmosphere. The vent opening 16 is located on a common wall as the sealing port 4, such that an adjacent coupled module 9a covers the vent opening 16. This obstruction restricts venting in the module 9 thereby maximizing negative pressure applied to the adjacent coupled module 9a. Obstructed venting may continue through a series of coupled modules, with the ultimate module 9 (e.g., module 9a) having the vent opening 16 be exposed, thereby, providing venting for the whole series.

## Claims

1. A module (9) for use in a combinatorial drug delivery device, the module formed to accommodate a drug vial (DV) with a septum, the module comprising:
a vial spike (1) formed to pierce the septum (S) of the drug vial (DV) with a free end of the vial spike (1) configured to be located interiorly of the septum (S), the vial spike (1) including an inlet path (2) open at the free end (11) and extending along the vial spike (1) to an inlet opening (12), and an outlet path (3) open at the free end and extending along the vial spike (1) to an outlet chamber (6), the outlet path (3) being separate from the inlet path (2);
a sealing port (4) selectively sealing the inlet opening (12);
a vent (10) in communication with the inlet path (2) between the inlet opening (12) and the free end (11);
a slidable piston valve (5) located in the outlet chamber (6), the piston valve (5) forming a seal in the outlet chamber (6) to define first and second chamber portions (6a, 6b), the outlet path (3) being in communication with the first chamber portion (6a);
an outlet fitting (7) in communication with the second chamber portion (6b); and,
a bypass passageway (8) extending between the outlet fitting (7) and an opening in the outlet chamber (6),
wherein, in an initial state, the piston valve is located in a first position where the first chamber portion is sealed from the bypass passageway, and,
wherein, with negative pressure introduced through the outlet fitting, the piston valve is caused to move to a second position where the first chamber portion is in communication with the bypass passageway,
wherein the vent (10) terminates at a vent opening (16), away from the inlet path (2), wherein the sealing port (4) and the vent opening (16) are located on a common wall of the module (9).

2. A module as in claim 1, wherein the vent (10) is a one-way vent which is normally closed and allows for gas flow into the inlet path (2).

3. A module as in claim 1, wherein the piston valve (5) includes a radial seal in sealing contact with a wall of the outlet chamber (6).

4. A combination comprising:
a first module (9) including:
a first vial spike (1) with a first free end, the first vial spike (1) including a first inlet path (2) open at the first free end and extending along the first vial spike (1) to a first inlet opening (12), and a first outlet path (3) open at the first free end and extending along the first vial spike (1) to a first outlet chamber (6), the first outlet path (3) being separate from the first inlet path (2);
a first sealing port (4) selectively sealing the first inlet opening (12);
a first vent (10) in communication with the first inlet path (2) between the first inlet opening (12) and the first free end;
a slidable first piston valve (5) located in the first outlet chamber (6), the first piston valve (5) forming a seal in the first outlet chamber (6) to define first and second primary chamber portions (6a, 6b), the first outlet path being in communication with the first primary chamber portion (6a);
a first outlet fitting (7) in communication with the second primary chamber portion (6b); and,
a first bypass passageway (8) extending between the first outlet fitting (7) and a first opening in the first outlet chamber (6),
a second module (9a) including:
a second vial spike (1a) with a second free end, the second vial spike (1a) including a second inlet path (2a) open at the second free end and extending along the second vial spike (1a) to a second inlet opening (12a), and a second outlet path (3a) open at the second free end and extending along the second vial spike (1a) to a second outlet chamber (6'), the second outlet path (3a) being separate from the second inlet path (2a);
a second sealing port (4a) selectively sealing the second inlet opening (12a);
a second vent (10a) in communication with the second inlet path (2a) between the second inlet opening (12a) and the second free end;
a slidable second piston valve (5a) located in the second outlet chamber (6'), the second piston valve (5a) forming a seal in the second outlet chamber (6') to define first and second secondary chamber portions (6'a, 6'b), the second outlet path (3a) being in communication with the first secondary chamber portion (6'a);
a second outlet fitting (7a) in communication with the second secondary chamber portion (6'b); and,
a second bypass passageway (8a) extending between the second outlet fitting (7a) and a second opening in the second outlet chamber (6'),
wherein, the second module (9a) is coupled to the first module (9) with the second outlet fitting (7a) extending through the first sealing port (4) to be in communication with the first inlet opening (12),
wherein, in an initial state, the first piston valve (5) is located in a first position where the first primary chamber portion (6a) is sealed from the first bypass passageway (8),
wherein, with negative pressure introduced through the first outlet fitting (7), the first piston valve (5) is caused to move to a second position where the first primary chamber portion (6a) is in communication with the first bypass passageway (8), and,
wherein, with the first piston valve (5) in the second position, the negative pressure is introduced to the second outlet fitting (7a) via the first outlet path (3) and the first inlet path (2),
wherein the first vent (10) terminates at a first vent opening (16), away from the first inlet path (2), wherein the first sealing port (4) and the vent opening (16) are located on a common wall of the first module (9), and,
wherein, with the second module (9a) coupled to the first module (9), the second module (9a) obstructs the first vent opening (16) to restrict venting therethrough.

5. A combination as in claim 4, wherein the second vent (10a) is exposed with the second sealing port (4a) sealing the second inlet opening (12a) with the second module (9a) coupled to the first module (9).

6. A combination as in claim 4, wherein, with the negative pressure introduced through the second outlet fitting (7a), the second piston valve (5a) is caused to move within the second outlet chamber (6') to cause the first secondary chamber portion (6'a) to come into communication with the second bypass passageway (8a).

## Patentansprüche

1. Modul (9) zur Verwendung in einer kombinatorischen Arzneimittelabgabevorrichtung, wobei das Modul dafür geformt ist, eine Arzneimittelampulle (DV) mit einem Septum aufzunehmen, wobei das Modul Folgendes umfasst:
eine Ampullenkanüle (1), die dafür geformt ist, das Septum (S) der Arzneimittelampulle (DV) mit einem freien Ende der Ampullenkanüle (1) zu durchstechen, das dafür konfiguriert ist, sich innerhalb des Septums (S) zu befinden, wobei die Ampullenkanüle (1) eine Einlassbahn (2), die an dem freien Ende (11) offen ist und sich entlang der Ampullenkanüle (1) bis zu einer Einlassöffnung (12) erstreckt, und eine Auslassbahn (3), die an dem freien Ende offen ist und sich entlang der Ampullenkanüle (1) bis zu einer Auslasskammer (6) erstreckt, einschließt, wobei die Auslassbahn (3) von der Einlassbahn (2) getrennt ist;
einen Abdichtungsanschluss (4), der die Einlassöffnung (12) selektiv abdichtet;
eine Lüftung (10) in Verbindung mit der Einlassbahn (2) zwischen der Einlassöffnung (12) und dem freien Ende (11);
ein verschiebbares Kolbenventil (5), das sich in der Auslasskammer (6) befindet, wobei das Kolbenventil (5) eine Dichtung in der Auslasskammer (6) bildet, um einen ersten und einen zweiten Kammerabschnitt (6a, 6b) zu definieren, wobei die Auslassbahn (3) in Verbindung mit dem ersten Kammerabschnitt (6a) steht;
ein Auslass-Anschlussstück (7) in Verbindung mit dem zweiten Kammerabschnitt (6b); und
einen Umgehungsdurchgang (8), der sich zwischen dem Auslass-Anschlussstück (7) und einer Öffnung in der Auslasskammer (6) erstreckt,
wobei sich, in einem anfänglichen Zustand, das Kolbenventil in einer ersten Position befindet, worin der erste Kammerabschnitt gegenüber dem Umgehungsdurchgang abgedichtet ist, und
wobei, mit Unterdruck, der durch das Auslass-Anschlussstück eingeleitet wird, das Kolbenventil dazu veranlasst wird, sich zu einer zweiten Position zu bewegen, worin der erste Kammerabschnitt in Verbindung mit dem Umgehungsdurchgang steht,
wobei die Lüftung (10) an einer Lüftungsöffnung (16) endet, weg von der Einlassbahn (2), wobei sich der Abdichtungsanschluss (4) und die Lüftungsöffnung (16) an einer gemeinsamen Wand des Moduls (9) befinden.

2. Modul nach Anspruch 1, wobei die Lüftung (10) eine Einweg-Lüftung ist, die normalerweise geschlossen ist und einen Gasstrom in die Einlassbahn (2) ermöglicht.

3. Modul nach Anspruch 1, wobei das Kolbenventil (5) eine radiale Dichtung in abdichtender Berührung mit einer Wand der Auslasskammer (6) einschließt.

4. Kombination, die Folgendes umfasst:
ein erstes Modul (9), das Folgendes einschließt:
eine erste Ampullenkanüle (1) mit einem ersten freien Ende, wobei die erste Ampullenkanüle (1) eine erste Einlassbahn (2), die an dem ersten freien Ende offen ist und sich entlang der ersten Ampullenkanüle (1) bis zu einer ersten Einlassöffnung (12) erstreckt, und eine erste Auslassbahn (3), die an dem ersten freien Ende offen ist und sich entlang der ersten Ampullenkanüle (1) bis zu einer ersten Auslasskammer (6) erstreckt, einschließt, wobei die erste Auslassbahn (3) von der ersten Einlassbahn (2) getrennt ist;
einen ersten Abdichtungsanschluss (4), der die erste Einlassöffnung (12) selektiv abdichtet;
eine erste Lüftung (10) in Verbindung mit der ersten Einlassbahn (2) zwischen der ersten Einlassöffnung (12) und dem ersten freien Ende;
ein verschiebbares erstes Kolbenventil (5), das sich in der ersten Auslasskammer (6) befindet, wobei das erste Kolbenventil (5) eine Dichtung in der ersten Auslasskammer (6) bildet, um einen ersten und einen zweiten primären Kammerabschnitt (6a, 6b) zu definieren, wobei die erste Auslassbahn in Verbindung mit dem ersten primären Kammerabschnitt (6a) steht;
ein erstes Auslass-Anschlussstück (7) in Verbindung mit dem zweiten primären Kammerabschnitt (6b); und
einen ersten Umgehungsdurchgang (8), der sich zwischen dem ersten Auslass-Anschlussstück (7) und einer ersten Öffnung in der ersten Auslasskammer (6) erstreckt,
ein zweites Modul (9a), das Folgendes einschließt:
eine zweite Ampullenkanüle (1a) mit einem zweiten freien Ende, wobei die zweite Ampullenkanüle (1a) eine zweite Einlassbahn (2a), die an dem zweiten freien Ende offen ist und sich entlang der zweiten Ampullenkanüle (1a) bis zu einer zweiten Einlassöffnung (12a) erstreckt, und eine zweite Auslassbahn (3a), die an dem zweiten freien Ende offen ist und sich entlang der zweiten Ampullenkanüle (1a) bis zu einer zweiten Auslasskammer (6') erstreckt, einschließt, wobei die zweite Auslassbahn (3a) von der zweiten Einlassbahn (2a) getrennt ist;
einen zweiten Abdichtungsanschluss (4a), der die zweite Einlassöffnung (12a) selektiv abdichtet;
eine zweite Lüftung (10a) in Verbindung mit der zweiten Einlassbahn (2a) zwischen der zweiten Einlassöffnung (12a) und dem zweiten freien Ende;
ein verschiebbares zweites Kolbenventil (5a), das sich in der zweiten Auslasskammer (6') befindet, wobei das zweite Kolbenventil (5a) eine Dichtung in der zweiten Auslasskammer (6') bildet, um einen ersten und einen zweiten sekundären Kammerabschnitt (6'a, 6'b) zu definieren, wobei die zweite Auslassbahn (3a) in Verbindung mit dem ersten sekundären Kammerabschnitt (6'a) steht;
ein zweites Auslass-Anschlussstück (7a) in Verbindung mit dem zweiten sekundären Kammerabschnitt (6'b); und
einen zweiten Umgehungsdurchgang (8a), der sich zwischen dem zweiten Auslass-Anschlussstück (7a) und einer zweiten Öffnung in der zweiten Auslasskammer (6') erstreckt,
wobei das zweite Modul (9a) an das erste Modul (9) gekoppelt ist, wobei sich das zweite Auslass-Anschlussstück (7a) durch den ersten Abdichtungsanschluss (4) erstreckt, um in Verbindung mit der ersten Einlassöffnung (12) zu stehen,
wobei sich, in einem anfänglichen Zustand, das erste Kolbenventil (5) in einer ersten Position befindet, worin der erste primäre Kammerabschnitt (6a) gegenüber dem ersten Umgehungsdurchgang (8) abgedichtet ist,
wobei, mit Unterdruck, der durch das erste Auslass-Anschlussstück (7) eingeleitet wird, das erste Kolbenventil (5) dazu veranlasst wird, sich zu einer zweiten Position zu bewegen, worin der erste primäre Kammerabschnitt (6a) in Verbindung mit dem ersten Umgehungsdurchgang (8) steht, und
wobei, mit dem ersten Kolbenventil (5) in der zweiten Position, der Unterdruck über die erste Auslassbahn (3) und die erste Einlassbahn (2) in das zweite Auslass-Anschlussstück (7a) eingeleitet wird,
wobei die erste Lüftung (10) an einer ersten Lüftungsöffnung (16) endet, weg von der ersten Einlassbahn (2), wobei sich der erste Abdichtungsanschluss (4) und die erste Lüftungsöffnung (16) an einer gemeinsamen Wand des ersten Moduls (9) befinden, und
wobei, mit dem an das erste Modul (9) gekoppelten zweiten Modul (9a), das zweite Modul (9a) die erste Lüftungsöffnung (16) blockiert, um das Entlüften durch diese hindurch zu beschränken.

5. Kombination nach Anspruch 4, wobei die zweite Lüftung (10a) freigelegt ist, wobei der zweite Abdichtungsanschluss (4a) die zweite Einlassöffnung (12a) abdichtet, wobei das zweite Modul (9a) an das erste Modul (9) gekoppelt ist.

6. Kombination nach Anspruch 4, wobei, mit dem Unterdruck, der durch das zweite Auslass-Anschlussstück (7a) eingeleitet wird, das zweite Kolbenventil (5a) dazu veranlasst wird, sich innerhalb der zweiten Auslasskammer (6') zu bewegen, um den ersten sekundären Kammerabschnitt (6'a) dazu zu veranlassen, in Verbindung mit dem zweiten Umgehungsdurchgang (8a) zu kommen.

## Revendications

1. Module (9) destiné à une utilisation dans un dispositif d'administration de médicaments combinatoire, le module étant formé pour accueillir un flacon de médicament (DV) avec un septum, le module comprenant :
un perforateur de flacon (1) formé pour percer le septum (S) du flacon de médicament (DV) avec une extrémité libre du perforateur de flacon (1) configuré pour être situé à l'intérieur du septum (S), le perforateur de flacon (1) incluant une voie d'entrée (2) ouverte au niveau de l'extrémité libre (11) et s'étendant le long du perforateur de flacon (1) jusqu'à une ouverture d'entrée (12), et une voie de sortie (3) ouverte au niveau de l'extrémité libre et s'étendant le long du perforateur de flacon (1) jusqu'à une chambre de sortie (6), la voie de sortie (3) étant séparée de la voie d'entrée (2) ;
un orifice d'étanchéité (4) scellant sélectivement l'ouverture d'entrée (12) ;
un évent (10) en communication avec la voie d'entrée (2) entre l'ouverture d'entrée (12) et l'extrémité libre (11) ;
une soupape à piston (5) coulissante située dans la chambre de sortie (6), la soupape à piston (5) formant un joint dans la chambre de sortie (6) pour définir des première et deuxième parties de chambre (6a, 6b), la voie de sortie (3) étant en communication avec la première partie de chambre (6a) ;
un raccord de sortie (7) en communication avec la deuxième partie de chambre (6b) ; et,
un passage de dérivation (8) s'étendant entre le raccord de sortie (7) et une ouverture dans la chambre de sortie (6),
dans lequel, dans un état initial, la soupape à piston est située dans une première position où la première partie de chambre est scellée vis-à-vis du passage de dérivation, et,
dans lequel, avec une pression négative introduite par le raccord de sortie, la soupape à piston est amenée à se déplacer vers une deuxième position où la première partie de chambre est en communication avec le passage de dérivation,
dans lequel l'évent (10) se termine au niveau d'une ouverture d'évent (16), à l'écart de la voie d'entrée (2), dans lequel l'orifice d'étanchéité (4) et l'ouverture d'évent (16) sont situés sur une paroi commune du module (9).

2. Module selon la revendication 1, dans lequel l'évent (10) est un évent unidirectionnel qui est normalement fermé et qui permet l'écoulement de gaz dans la voie d'entrée (2).

3. Module selon la revendication 1, dans lequel la soupape à piston (5) inclut un joint radial en contact étanche avec une paroi de la chambre de sortie (6).

4. Combinaison comprenant :
un premier module (9) incluant :
un premier perforateur de flacon (1) avec une première extrémité libre, le premier perforateur de flacon (1) incluant une première voie d'entrée (2) ouverte au niveau de la première extrémité libre et s'étendant le long du premier perforateur de flacon (1) jusqu'à une première ouverture d'entrée (12), et une première voie de sortie (3) ouverte à la première extrémité libre et s'étendant le long du premier perforateur de flacon (1) jusqu'à une première chambre de sortie (6), la première voie de sortie (3) étant séparée de la première voie d'entrée (2) ;
un premier orifice d'étanchéité (4) scellant sélectivement la première ouverture d'entrée (12) ;
un premier évent (10) en communication avec la première voie d'entrée (2) entre la première ouverture d'entrée (12) et la première extrémité libre ;
une première soupape à piston (5) coulissante située dans la première chambre de sortie (6), la première soupape à piston (5) formant un joint dans la première chambre de sortie (6) pour définir des première et deuxième parties de chambre primaires (6a, 6b), la première voie de sortie étant en communication avec la première partie de chambre primaire (6a) ;
un premier raccord de sortie (7) en communication avec la deuxième partie de chambre primaire (6b) ; et,
un premier passage de dérivation (8) s'étendant entre le premier raccord de sortie (7) et une première ouverture dans la première chambre de sortie (6),
un deuxième module (9a) incluant :
un deuxième perforateur de flacon (1a) avec une deuxième extrémité libre, le deuxième perforateur de flacon (1a) incluant une deuxième voie d'entrée (2a) ouverte au niveau de la deuxième extrémité libre et s'étendant le long du deuxième perforateur de flacon (1a) jusqu'à une deuxième ouverture d'entrée (12a), et une deuxième voie de sortie (3a) ouverte au niveau de la deuxième extrémité libre et s'étendant le long du deuxième perforateur de flacon (1a) jusqu'à une deuxième chambre de sortie (6'), la deuxième voie de sortie (3a) étant séparée de la deuxième voie d'entrée (2a) ;
un deuxième orifice d'étanchéité (4a) scellant sélectivement la deuxième ouverture d'entrée (12a) ;
un deuxième évent (10a) en communication avec la deuxième voie d'entrée (2a) entre la deuxième ouverture d'entrée (12a) et la deuxième extrémité libre ;
une deuxième soupape à piston (5a) coulissante située dans la deuxième chambre de sortie (6'), la deuxième soupape à piston (5a) formant un joint dans la deuxième chambre de sortie (6') pour définir des première et deuxième parties de chambre secondaires (6'a, 6'b), la deuxième voie de sortie (3a) étant en communication avec la première partie de chambre secondaire (6'a) ;
un deuxième raccord de sortie (7a) en communication avec la deuxième partie de chambre secondaire (6'b) ; et,
un deuxième passage de dérivation (8a) s'étendant entre le deuxième raccord de sortie (7a) et une deuxième ouverture dans la deuxième chambre de sortie (6'),
dans laquelle le deuxième module (9a) est couplé au premier module (9) avec le deuxième raccord de sortie (7a) s'étendant à travers le premier orifice d'étanchéité (4) pour être en communication avec la première ouverture d'entrée (12),
dans laquelle, dans un état initial, la première soupape à piston (5) est située dans une première position où la première partie de chambre primaire (6a) est scellée vis-à-vis du premier passage de dérivation (8),
dans laquelle, une pression négative étant introduite par le premier raccord de sortie (7), la première soupape à piston (5) est amenée à se déplacer vers une deuxième position où la première partie de chambre primaire (6a) est en communication avec le premier passage de dérivation (8), et,
dans laquelle, la première soupape à piston (5) étant dans la deuxième position, la pression négative est introduite dans le deuxième raccord de sortie (7a) *via* la première voie de sortie (3) et la première voie d'entrée (2),
dans laquelle le premier évent (10) se termine au niveau d'une première ouverture d'évent (16), à l'écart de la première voie d'entrée (2), dans laquelle le premier orifice d'étanchéité (4) et la première ouverture d'évent (16) sont situés sur une paroi commune du premier module (9), et,
dans laquelle, avec le deuxième module (9a) couplé au premier module (9), le deuxième module (9a) obstrue la première ouverture d'évent (16) pour restreindre la ventilation à travers celle-ci.

5. Combinaison selon la revendication 4, dans laquelle le deuxième évent (10a) est exposé, le deuxième orifice d'étanchéité (4a) scellant la deuxième ouverture d'entrée (12a), le deuxième module (9a) étant couplé au premier module (9).

6. Combinaison selon la revendication 4, dans laquelle, avec la pression négative introduite par le deuxième raccord de sortie (7a), la deuxième soupape à piston (5a) est amenée à se déplacer à l'intérieur la deuxième chambre de sortie (6') pour amener la première partie de chambre secondaire (6'a) à entrer en communication avec le deuxième passage de dérivation (8a).
